# Europäisches Patentamt

## European Patent Office

## Office européen des brevets

(19)

(11) Publication number: **0 287 152 B1**

(12) **EUROPEAN PATENT SPECIFICATION**

(45) Date of publication of patent specification: **09.12.92** (51) Int. Cl.[5]: **C12P 13/04**, A23K 1/16

(21) Application number: **88200630.7**

(22) Date of filing: **05.04.88**

(54) Method for preparation or extracting amino acids from manure.

(30) Priority: **06.04.87 NL 8700803**

(43) Date of publication of application:
**19.10.88 Bulletin 88/42**

(45) Publication of the grant of the patent:
**09.12.92 Bulletin 92/50**

(84) Designated Contracting States:
**AT BE CH DE ES FR GB GR IT LI LU NL SE**

(56) References cited:
**DE-A- 2 535 296**
**FR-A- 2 119 567**
**US-A- 3 838 199**
**US-A- 4 104 124**
**US-A- 4 292 328**

**BIOTECHNOLOGY AND BIOENGINEERING, vol. 21, no. 1, 1979, pages 19-38, J. Wiley & Sons, Inc.; M.L. SHULER et al.: "Process for the aerobic conversion of poultry manure into high-protein feedstuff"**

**CHEMICAL ABSTRACTS, vol. 103, no. 3, July 1985, page 466, no. 21213g, Columbus, Ohio, US; & JP-A-60 47 691 (TORAY INDUSTRIES INC.) 15-03-1985**

(73) Proprietor: **GIST-BROCADES N.V.**
**Wateringseweg 1**
**NL-2611 XT Delft(NL)**

(72) Inventor: **Slijkhuis, Harmen**
**Molenvlietbrink 124**
**NL-3448 HP Woerden(NL)**
Inventor: **Sanders, Johan Pieter Marinus**
**Thorbeckestraat 73**
**NL-2613 BV Delft(NL)**

(74) Representative: **Huygens, Arthur Victor, Dr. et al**
**c/o Gist-Brocades N.V. Patents & Trademarks Department Wateringseweg 1 PO Box 1**
**NL-2600 MA Delft(NL)**

CHEMICAL ABSTRACTS, vol. 100, no. 5, January 1984, page 351, no. 33272b, Columbus, Ohio, US; & CS-A-201812 (M. BUCKO et al.) 15-02-1983

CHEMICAL ABSTRACTS, vol. 93, no. 15, 13th October 1980, page 599, no. 148746p, Columbus, Ohio, US; M.J. GINNIVAN et al.: "Evaluation of the thermophilically treated pig slurry as a source of nutrients in pig diets", & AGRIC. ENVIRON. 1980, 5(3), 227-39

CHEMICAL ABSTRACTS, vol. 93, no. 5, 4th August 1980, page 798, no. 45112z, Columbus, Ohio, US; S. HAYASHIDA: "Fertilizer and fodder production from cattle waste by fermentation", & KANKYO KAGAKU TOKUBETSU KENKYU KENKYU HOOKUSHU 1980, B44-R33-3, SHIGEN JUNKAN, 12-15

## Description

The invention relates to a method for preparing or extracting amino acids, in particular L-amino acids such as L-lysine (hereinafter referred to as lysine) and L-methionine (hereinafter referred to as methionine) from manure, in particular from semi-liquid pig manure.

In our Western community life, animal excrements form an ever more pressing problem. Intensive livestock breeding, in particular pig farming, produces large quantities of manure which cannot be adequately disposed of in agriculture in the immediate vicinity. Transportation to outlets at a greater distance from the points of production involves high costs. Another possible way of solving the manure problem or, at any rate, keeping it in check, is a suitable treatment of the manure, the manure being converted into products which are not harmful or are less harmful to the environment or which may possibly even be used beneficially. In this connection, it is worth considering, for example, anaerobic fermentation, aerobic stabilization and production of biomass.

Anaerobic fermentation yields a methane-rich gas which can be used to cover the cost of said process wholly or partially. However, this does not solve the nitrogen problem and other problems associated with the manure problem, such as the phosphate problem. The effluent from the anaerobic purification still contains the nitrogen, principally in the form of ammonia and, moreover, phosphates and copper salts.

Aerobic stabilization is relatively expensive because of the oxygen requirement of the process. The nitrogen is partially fixed in the form of cell material. The sludge produced may in certain cases be used as an animal fodder constituent.

The production of biomass from manure by means of protein-rich microoganisms, for example yeasts, is a variant of aerobic stabilization in which selected microorganisms are used. It is a relatively expensive process. The product (the sludge) has a certain nutritional value but the content of useful nutrients is relatively low so that its use is not attractive and, in certain cases such as, for example modern factory farming, is even prohibitive for that reason. If it is intended to obtain a product with a higher beneficial nutritional value in the production of biomass from manure, the manure will first have to be separated, for example, into the water-soluble and the water-insoluble portion, and the biomass production will have to be carried out with the water-soluble portion in order, in this manner, to achieve separation of the valuable biomass from the solids which contain little nutritional value.

US-A-4,292,328 describes a thermophilic aerobic digestion process in which manure is digested to various digested products, including single cell proteins. The naturally occurring microorganisms are responsible for the microbial action in the digestion process. When extra microorganisms are inoculated, the normal thermophilic digestion process continues.

The bulk of pig feed consists of cereal, soya, tapioca and other carbohydrate and protein sources. The proteins in cereals are relatively poor in the essential amino acids lysine, methionine, threonine and tryptophan. In addition to certain vitamins and growth-promoting substances, these amino acids are therefore often added to the feed. The amino acids necessary for this are usually produced microbiologically in the L-form using a suitable microorganism on a nutrient medium specially made up for this purpose (see, for example, FR-A-2,541,867 and US-A-3,729,381). Chemical synthesis is also possible; but then we have to deal with stereoisomer problems which tend to increase the cost price. Chemical synthesis usually results in racemates so that racemate resolution is necessary in order to prepare an optically pure amino acid.

Surprisingly, it has now been found that certain valuable amino acids, such as lysine and methionine, can be produced microbiologically using manure as the nutrient medium.

The invention therefore relates to a method for the preparation or extraction of valuable amino acids, such as the essential amino acids lysine and methionine, from manure of domesticated farm animals (pigs, cattle and poultry) by pasteurization or sterilisation of the manure followed by inoculating said manure with bacteria which are capable of producing the amino acids mentioned and allowing the bacteria to grow in the manure, after which the amino acids mentioned are isolated from the mixture. Other essential amino acids that can be used in feed like isoleucine, tryptophan and other amino acids shall be included in this invention.

Bacteria which are capable of producing the amino acids referred to belong to the genera Arthrobacter, Bacillus, Brevibacterium, Corynebacterium, Escherichia, Microbacterium, Micrococcus and Pseudomonas. In particular, species belonging to the group of "coryneform" bacteria consisting of Arthrobacter, Brevibacterium and Corynebacterium, appear to be particularly suitable. Suitable species are, for example, Brevibacterium flavum, Brevibacterium lactofermentum, Corynebacterium glutamicum, and Arthrobacter SP B-1. Specific examples for the preparation of lysine and methionine are species with the following deposit numbers: Brevibacterium flavum ATCC 21475, Brevibacterium flavum ATCC 21518, Brevibacterium lactofermentum ATCC 21798, Corynebacterium glutamicum ATCC 21608, Corynebacterium glutamicum ATCC 21516, Arthrobacter SP B-1 ATCC 21859.

Manure from domestic farm animals appears to be particularly suitable for use in the method according to the invention. Semi-liquid pig manure appears to give excellent results. The semi-liquid manure is first preferably pasteurized or sterilized to prevent undesirable growth of microorganisms and to kill pathogenic microorganisms. The method according to the invention may optionally also be carried out with the so-called "supernatant" of semi-liquid manure, that is to say, with the aqueous liquid which is obtained by decanting after settling or by centrifuging semi-liquid manure, and which contains all the water-soluble constituents of semi-liquid manure.

The composition of manure may vary within wide limits and is dependent, inter alia, on the type of animal from which the manure originates (for example, pigs, cattle or poultry), and on the manner in which the manure is collected. Semi-liquid manure from pigs or cows usually has a dry substance content of less than 10% by weight, usually 8-9% by weight; the organic substance content is usually in the region of 5% by weight; the total nitrogen content varies from 0.35 to 0.65% by weight, and the phosphate content is 0.4-0.5% by weight ($P_2O_5$). "More solid" manure has a moisture content of 65-85% by weight, an organic substance content of 10-20% by weight, a total nitrogen content of 0.6 to 1.0% by weight and a phosphate content ($P_2O_5$) of 0.5 to 0.9% by weight.

In view of the C/N ratio of semi-liquid manure, it is advisable to add a certain quantity of a carbon source to semi-liquid manure in order to utilize the nitrogen present as ideally and completely as possible. Molasses, glucose or sucrose, for example, may be used as the carbon source to be added. This method also makes it possible to use sugar-containing waste products such as beet pulp and, to be specific, in a quantity which is equivalent to up to 200 g of glucose per kg of manure, preferably less than 100 g of glucose equivalents per kg of manure.

If the method is carried out with the total manure and not just with the "supernatant", it may be beneficial to add certain enzymes to the manure and in particular, starch-splitting and/or cellulose-splitting enzymes, for example amylase and/or cellulase. In this manner, a greater portion of the carbon source present in the manure can be beneficially used for the microbiological synthesis of the amino acids. The addition of such enzymes may even render the addition of a carbon source to the manure referred to above superfluous. It is also possible in this manner to add a cheap starch product or cellulose-containing material (for example, agricultural waste) to the manure in addition to, or instead of, the sugars referred to above as a carbon source. In this manner, the efficiency of the method can be increased still further and the manure problem reduced to minimum proportions. The method described makes it possible to allow the enzymes to act before, but preferably during, the fermentation process. Savings on enzyme costs can further be made by starting from microorganisms which are themselves capable of amylase, pectinase, hemicellulase and/or cellulase production, possibly after cloning the necessary genes.

The amino acids produced by the method according to the invention are present in the reaction mixture in dissolved form. The working up therefore presents few problems. The solid constituents are first separated from the slurry, for example by centrifuging or filtration. The amino acid can be isolated from the aqueous phase, for example, by means of a suitable ion exchanger such as a strongly acidic cation exchanger in the H form or the NH$_4$ form. The amino acid can be obtained in pure form by elution in the usual manner from the ion exchanger. The aqueous liquid which contains the amino acid produced may also be concentrated without further purification, for example by means of a membrane process or by concentration by freezing. If desired, the amino acid can additionally be isolated in pure form from the concentrate, which contains, in addition to the amino acid produced by the method according to the invention, also water-soluble constituents of manure which are not consumed in the method, such as phosphates, and sodium, potassium and copper salts. Said amino acid can then be used as an animal feed additive. However, the concentrate may also be used without further purification as an animal feed additive, if necessary after removal of toxic substances.

Generally, copper occurs in manure because copper salts are added to animal feed as a growth promoter. It is not itself stored in the body but ends up in the manure and usually, therefore, in the environment. As a result of using the concentrate as an animal feed additive, copper is recirculated. There is then no need, or less need, for copper to be added to the feed and the environment suffers less copper pollution.

The phosphate is present in animal feed partly in the form of phytin which originates, for example, from soya. The domestic farm animal is insufficiently capable of utilizing the phosphate in phytin. Although the phosphate which is liberated in the small intestine from phytin can still be absorbed by the animal, the phosphate released from phytin in the large intestine can no longer be absorbed so that said phosphate, together with unconverted phytin, ends up in the manure. Recirculation of said phosphate and the unconverted phytin via the abovementioned concentrate can already also result in a reduction of the phosphate pollution in the environment. Less phosphate then has to be added to the feed than is the case in the present feeding method.

Phytin can be converted by the phytase enzyme into phosphate which can be absorbed by the animal. If, therefore, the phosphate is liberated by means of phytase from the phytin present in the manure before, during or after the production of amino acids from manure according to the present method and said phosphate is recirculated, for example via the abovementioned concentrate, the phosphate pollution of the environment is reduced still further. A particular embodiment of the method according to the invention is therefore one in which the phosphate is liberated from phytin by means of phytase before, during or after the production of essential amino acids from manure an is recirculated in this form with the other nutrients. The unlocking of the phytin phosphate is preferably carried out during the production of amino acid. For this purpose, phytase is added to the reaction mixture and, to be specific, in a quantity which is sufficient to break down the phytin. If a microorganism which makes sufficient phytase is present during the fermentation, the addition may be omitted. For example, use may be made of micoorganisms which, after cloning of the necessary genes, are themselves capable of producing phytase. The abovementioned concentrate which can be obtained as the final result of said method then contains, in addition to the amino acid produced, not only copper which is used as a feed additive, but also phosphate in completely "liberated" form. Use of said concentrate as an animal feed additive results in an appreciable reduction of the pollution of the environment with nitrogen, copper and phosphate.

If the aqueous phase of the slurry is concentrated by a membrane process, for example by means of ultrafiltration, waste water is obtained, which can be discharged directly into the public water system.

The method according to the invention is expediently carried out at a temperature of 10-70°C and is preferably carried out at a temperature of 15-50°C, a retention of 24-144 hours usually being sufficient.

Using the method according to the invention, it is possible to produce a valuable animal feed additive from a troublesome waste product. Whatever is left of the semi-liquid manure used in addition to the amino acid produced or the concentrate obtained (filtered manure constituents, aqueous liquids) presents much less of a problem than the original semi-liquid manure. The solid residues mentioned can be used as manure material or soil improver in agriculture and horticulture. Transportation costs present less of a problem as a result of the smaller volume. The content of environmental pollutants such as phosphate, copper and nitrogen, is also lower than that of the original manure so that the solid residues produced by said process from more pigs can be deposited more densely on the land in the production operation without giving rise to environmental overloading. The aqueous liquids which remain can often be discharged directly. Depending on the working-up process, they may, if necessary, be further processed in a biological purification plant.

The present method is capable (dependent to some extent on the chosen microorganism) of producing 0.5-15 g of amino acid per kg of semi-liquid manure or 0.2-8 g of amino acid per kg of supernatant thereof. The higher yields can be achieved on adding an additional carbon source to the reaction mixture. Yet higher yields can be obtained after adding an additional nitrogen source to the manure.

Example I

Arthrobacter SP B-1 (ATCC 21859), which is capable of producing lysine, was cultivated for 24 hours at 30°C in BHI (Difco) and then used to inoculate 1.5 kg of semi-liquid pig manure supernatant, which had been sterilized beforehand (45 min, 120°C), at a level of 2%.

The supernatant had a reducing substance content (expressed in glucose units) of 1.2 g/kg, a total nitrogen content of 2.7 g/kg and an ammonium content of 2.1 g/kg.

The culture was aerated and stirred. After 144 hours, the fermentation was stopped and the reaction mixture was worked up in the usual manner. The cell debris was removed in a centrifuge and lysine was isolated from the aqueous phase by means of an ion exchanger (Amberlite IR-120 in H form). Yield: 0.9 g of lysine.

Example II

Example I was repeated with the difference that 30 g of glucose was added to the supernatant after sterilization. Yield: 4.2 g of lysine.

Example III

A quantity of molasses which is equivalent to 4 kg of glucose was added to 100 kg of semi-liquid pig manure in a fermenter. The semi-liquid manure contained 3.7 g of reducing substance, expressed in glucose units, 5.5 g of total nitrogen, 4.3 g of ammonium nitrogen, 4.2 g of $P_2O_5$ and 55 mg of copper per kg. After sterilization at pH = 6 (1 hour, 120°C) the fermenter was inoculated with 1 litre of Brevibacterium flavum (ATCC 21475) which is capable of producing lysine. The pH was adjusted by addition of 2N $H_2SO_4$ or 50% w/w NaOH. The B. flavum culture was obtained by cultivation in a medium containing 10 g of yeast extract per litre and 20 g of glucose per litre at 30°C and pH = 7.5 for 24 hours. The mixture in the fermenter was aerated and stirred for 72 hours at 30°C and pH = 7.5, after which the fermentation was stopped and the slurry was worked up. The solid constituents were separated from the aqueous phase in a centrifuge. The aqueous phase was concentrated by means of ultrafiltration to a volume of 10 litres. The concentrate obtained, which contained 0.6 kg of lysine, was used as an animal feed additive.

Example IV

Example III was repeated with the difference that 2.5 g of phytase (Sigma, batch no. 61F-8025) were also added to the starting mixture. A concentrate was obtained which contained 0.6 kg of lysine, 0.15 kg of "consumable" phosphorus and 510 mg of copper. The consumable phosphorous was added as free inorganic phosphates.

Example V

The Examples I and II were repeated with the difference that, if Corynebacterium glutamicum (ATCC 21608), which is capable of producing methionine, was used as the microorganism, 0.5 and 2.1 g respectively of methionine were obtained.

**Claims**

1.  Method for preparing or extracting amino acids, in particular L-amino acids such as L-lysine and L-methionine, from manure, characterized in that the manure of domestic farm animals is pasteurized or sterilized and subsequently is inoculated with bacteria which are capable of producing the amino acids mentioned, the bacteria are allowed to grow in the manure and the reaction mixture is worked up after the fermentation.

2.  Method according to Claim 1, characterized in that this is carried out with semi-liquid pig manure.

3.  Method according to Claim 1 or 2, characterized in that this is carried out with the supernatant of the semi-liquid manure.

4.  Method according to any one of Claims 1-3, characterized in that this is carried out at a temperature of 10-70°C, preferably at a temperature of 15-50°C, with a retention time of 24-144 hours.

5.  Method according to any one of Claims 1-4, characterized in that a carbon source such as molasses, glucose, sucrose, beet pulp or another sugar-containing agricultural waste is added to the semi-liquid manure.

6.  Method according to Claim 5, characterized in that a carbon source is added in a quantity of less than 200 g of glucose equivalents per kg of manure, preferably less than 100 g of glucose equivalents per kg of manure.

7.  Method according to any one of Claims 1-6, characterized in that the yield of L-lysine after the fermentation is at least 1 g per litre of manure or 0.5 g per kg of supernatant.

8.  Method according to any one of Claims 1-6, characterized in that the yield of L-methionine after the fermentation is at least 0.5 g per litre of manure or 0.2 g per kg of supernatant.

9.  Method according to any one of Claims 1-8, characterized in that species of the genera Arthrobacter, Bacillus, Brevibacterium, Corynebacterium, Escherichia, Microbacterium, Micrococcus or Pseudomonas are used as bacteria.

10. Method according to Claim 9, characterized in that species belonging to the group of "coryneforme" bacteria, consisting of Arthrobacter, Brevibacterium and Corynebacterium are used as bacteria.

11. Method according to Claim 10, characterized in that Brevibacterium flavum, Brevibacterium lactofermentum, Corynebacterium glutamicum or Arthrobacter SP B-1 are used as species.

12. Method according to any one of Claims 9-11, characterized in that strains having the following deposit numbers are used: ATCC 21475, ATCC 21518, ATCC 21798, ATCC 21608, ATCC 21516 or ATCC 21859.

13. Method according to any one of Claims 1-12, characterized in that a starch-splitting and/or cellulose-splitting enzyme is added to the reaction mixture.

14. Method according to Claim 13, characterized in that a material containing a starch source and/or cellulose is added to the reaction mixture.

15. Method according to any one of Claims 1-14, characterized in that the enzyme phytase is added to the reaction mixture in a quantity of 10-50 mg per kg of manure.

16. Method according to any one of Claims 1-15, characterized in that the reaction mixture is worked up by first separating off the solid constituents by centrifuging or filtration and then extracting the amino acid from the aqueous phase or the filtrate by means of a ion exchanger.

17. Method according to Claim 16, characterized in that a strongly acid cation exchanger in the H form, from which the amino acid is extracted by elution, is used as ion exchanger.

18. Method according to any one of Claims 1-17, characterized in that the reaction mixture is worked up by first separating off the solid constituents by centrifuging or filtration and then concentrating the aqueous phase via a membrane process or by concentration by freezing.

**Patentansprüche**

1. Verfahren zur Herstellung oder Extraktion von Aminosäuren, insbesondere L-Aminosäuren, wie L-Lysin und L-Methionin, aus Mist, dadurch gekennzeichnet, dass der Mist von domestizierten Nutztieren pasteurisiert oder sterilisiert und anschliessend mit Bakterien, die die erwähnten Aminosäuren zu erzeugen vermögen, geimpft wird, wobei man die Bakterien in dem Mist wachsen lässt und das Reaktionsgemisch nach der Fermentierung aufgearbeitet wird.

2. Verfahren nach Anspruch 1, dadurch gekennzeichnet, dass dieses mit halbflüssigem Schweinemist ausgeführt wird.

3. Verfahren nach Anspruch 1 oder 2, dadurch gekennzeichnet, dass dieses mit der überstehenden Flüssigkeit des halbflüssigen Mists ausgeführt wird.

4. Verfahren nach einem der Ansprüche 1 bis 3, dadurch gekennzeichnet, dass dieses bei einer Temperatur von 10 bis 70°C, vorzugsweise bei einer Temperatur von 15 bis 50°C, mit einer Verweilzeit von 24 bis 144 Stunden ausgeführt wird.

5. Verfahren nach einem der Ansprüche 1 bis 4, dadurch gekennzeichnet, dass eine Kohlenstoffquelle, wie Melasse, Glucose, Saccharose, ausgelaugte Zuckerrübenschnitzel oder ein anderer zuckerhaltiger landwirtschaftlicher Abfall, zu dem halbflüssigen Mist zugesetzt wird.

6. Verfahren nach Anspruch 5, dadurch gekennzeichnet, dass eine Kohlenstoffquelle in einer Menge von weniger als 200 g Glucoseäquivalenten pro kg Mist, vorzugsweise weniger als 100 g Glucoseäquivalenten pro kg Mist, zugesetzt wird.

7. Verfahren nach einem der Ansprüche 1 bis 6, dadurch gekennzeichnet, dass die Ausbeute an L-Lysin nach der Fermentierung mindestens 1 g pro 1 Mist oder 0,5 g pro kg überstehende Flüssigkeit beträgt.

8. Verfahren nach einem der Ansprüche 1 bis 6, dadurch gekennzeichnet, dass die Ausbeute an L-Methionin nach der Fermentierung mindestens 0,5 g pro 1 Mist oder 0,2 g pro kg überstehende Flüssigkeit beträgt.

9. Verfahren nach einem der Ansprüche 1 bis 8, dadurch gekennzeichnet, dass Spezies der Gattungen Arthrobacter, Bacillus, Brevibacterium, Corynebacterium, Escherichia, Microbacterium, Micrococcus oder Pseudomonas als Bakterien verwendet werden.

10. Verfahren nach Anspruch 9, dadurch gekennzeichnet, dass Spezies, die zu der Gruppe der "coryneformen" Bakterien, die aus Arthrobacter, Brevibacterium und Corynebacterium besteht, gehören, als Bakterien verwendet werden.

11. Verfahren nach Anspruch 10, dadurch gekennzeichnet, dass Brevibacterium flavum, Brevibacterium lactofermentum, Corynebacterum glutamicum oder Arthrobacter SP B-1 als Spezies verwendet werden.

12. Verfahren nach einem der Ansprüche 9 bis 11, dadurch gekennzeichnet, dass Stämme mit den folgenden Hinterlegungsnummern verwendet werden: ATCC 21475, ATCC 21518, ATCC 21798, ATCC 21608, ATCC 21516 oder ATCC 21859.

13. Verfahren nach einem der Ansprüche 1 bis 12, dadurch gekennzeichnet, dass ein stärkespaltendes und/oder cellulosespaltendes Enzym zu dem Reaktionsgemisch zugesetzt wird.

**14.** Verfahren nach Anspruch 13, dadurch gekennzeichnet, dass ein Material, das eine stärkequelle und/oder Cellulosequelle enthält, zu dem Reaktionsgemisch zugesetzt wird.

**15.** Verfahren nach einem der Ansprüche 1 bis 14, dadurch gekennzeichnet, dass das Enzym Phytase in einer Menge von 10 bis 50 mg pro kg Mist zu dem Reaktionsgemisch zugesetzt wird.

**16.** Verfahren nach einem der Ansprüche 1 bis 15, dadurch gekennzeichnet, dass das Reaktionsgemisch aufgearbeitet wird, indem man zuerst die festen Bestandteile durch Zentrifugieren oder Filtration abtrennt und dann die Aminosäure aus der wässrigen Phase oder dem Filtrat mit Hilfe eines Ionenaustauschers extrahiert.

**17.** Verfahren nach Anspruch 16, dadurch gekennzeichnet, dass ein stark saurer Kationenaustauscher in der H-Form, aus dem die Aminosäure durch Eluieren extrahiert wird, als Ionenaustauscher verwendet wird.

**18.** Verfahren nach einem der Ansprüche 1 bis 17, dadurch gekennzeichnet, dass das Reaktionsgemisch aufgearbeitet wird, indem man zuerst die festen Bestandteile durch Zentrifugieren oder Filtration abtrennt und dann die wässrige Phase über einen Membranprozess oder durch Einengen durch Gefrieren einengt.

**Revendications**

**1.** Procédé de préparation ou d'extraction d'acides aminés, en particulier de L-acides aminés tels que la L-lysine et la L-méthionine, à partir de fumier, caractérisé en ce que le fumier d'animaux domestiques d'élevage est pasteurisé ou stérilisé et est ensuite inoculé avec des bactéries qui sont capables de produire les acides aminés mentionnés, les bactéries sont mises à croître dans le fumier et le mélange de réaction est fractionné après la fermentation.

**2.** Procédé suivant la revendication 1, caractérisé en ce qu'il est exécuté avec du fumier de porc semi-liquide.

**3.** Procédé suivant la revendication 1 ou 2, caractérisé en ce qu'il est exécuté avec le surnageant du fumier semi-liquide.

**4.** Procédé suivant l'une quelconque des revendications 1 à 3, caractérisé en ce qu'il est exécuté à une température de 10 à 70°C, de préférence à une température de 15 à 50°C, avec un temps de séjour de 24 à 144 heures.

**5.** Procédé suivant l'une quelconque des revendications 1 à 4, caractérisé en ce qu'une source de carbone, comme de la mélasse, du glucose, du saccharose, de la pulpe de betterave ou un autre déchet agricole contenant du sucre, est ajoutée au fumier semi-liquide.

**6.** Procédé suivant la revendication 5, caractérisé en ce qu'une source de carbone est ajoutée en une quantité de moins de 200 g d'équivalents de glucose par kg de fumier, de préférence de moins de 100 g d'équivalents de glucose par kg de fumier.

**7.** Procédé suivant l'une quelconque des revendications 1 à 6, caractérisé en ce que la production de L-lysine après la fermentation est d'au moins 1 g par litre de fumier ou 0,5 g par kg de surnageant.

**8.** Procédé suivant l'une quelconque des revendications 1 à 6, caractérisé en ce que la production de L-méthionine après la fermentation est d'au moins 0,5 g par litre de fumier ou 0,2 kg par kg de surnageant.

**9.** Procédé suivant l'une quelconque des revendications 1 à 8, caractérisé en ce qu'on utilise comme bactéries des espèces des genres Arthrobacter, Bacillus, Brevibacterium, Corynebacterium, Escherichia, Microbacterium, Micrococcus ou Pseudomonas.

**10.** Procédé suivant la revendication 9, caractérisé en ce qu'on utilise comme bactéries des espèces appartenant au groupe des bactéries "corynéformes" formé par Arthrobacter, Brevibacterium et Corynebacterium.

**11.** Procédé suivant la revendication 10, caractérisé en ce qu'on utilise comme espèce Brevibacterium flavum, Brevibacterium lactofermentum, Corynebacterium glutamicum ou Arthrobacter SP B-1.

**12.** Procédé suivant l'une quelconque des revendications 9 à 11, caractérisé en ce qu'on utilise les souches ayant les numéros de dépôt suivants : ATCC 21475, ATCC 21518, ATCC 21798, ATCC 21608, ATCC 21516 ou ATCC 21589.

**13.** Procédé suivant l'une quelconque des revendications 1 à 12, caractérisé en ce qu'on ajoute au mélange de réaction une enzyme décomposant l'amidon et/ou décomposant la cellulose.

14. Procédé suivant la revendication 13, caractérisé en ce qu'on ajoute au mélange de réaction une matière contenant une source d'amidon et/ou de la cellulose.

15. Procédé suivant l'une quelconque des revendications 1 à 14, caractérisé en ce qu'on ajoute l'enzyme phytase au mélange de réaction en une quantité de 10 à 50 mg par kg de fumier.

16. Procédé suivant l'une quelconque des revendications 1 à 15, caractérisé en ce qu'on fractionne le mélange de réaction en séparant d'abord les constituants solides par centrifugation ou filtration, puis en extrayant l'acide aminé de la phase aqueuse ou du filtrat à l'aide d'un échangeur d'ions.

17. Procédé suivant la revendication 16, caractérisé en ce qu'on utilise comme échangeur d'ions un échangeur de cations fortement acide sous la forme H à partir duquel on isole l'acide aminé par élution.

18. Procédé suivant l'une quelconque des revendications 1 à 17, caractérisé en ce qu'on fractionne le mélange de réaction en séparant d'abord les constituants solides par centrifugation ou filtration, puis en concentrant la phase aqueuse par une opération avec membrane ou par concentration avec congélation.